# EUROPEAN PATENT APPLICATION

(11) **EP 4 088 714 A1**
(43) Date of publication of application: **16.11.2022**
(21) Application number: 21460025.6
(22) Date of filing: 10.05.2021
(51) Int. Cl.: A61K 9/00, A61K 47/12, A61K 31/46, A61P 27/10

(54) **OPHTHALMIC PHARMACEUTICAL COMPOSITION COMPRISING ATROPINE**

(71) Applicant: Warszawskie Zaklady Farmaceutyczne Polfa S.A., 01-207 Warszawa (PL)
(72) Inventor: PIETRZAK, Tomasz, 95-020 Wisniowa Gora (PL); CEBULSKA, Ewelina, 05-650 Chynow (PL); LUCZYNSKI, Pawel, 02-776 Warszawa (PL); MAGNUSZEWSKA, Monika, 05-806 Nowa Wies Warszawska (PL)
(74) Representative: Kondrat, Mariusz

(57) **Abstract**

The present invention is related to a preservative free ophthalmic low concentration atropine sulfate composition containing atropine sulfate in an amount from about 0.005 w/w% to about 0.05 w/w%, citrate buffer, a tonicity agent and water, having a pH from about 4.0 to about 5.5. Optionally this composition can also contain other excipients such as a pH adjuster, a chelating agent, a complexing agent, and/or a viscosity agent. The invention also relates to a process for the preparation of this composition and its use in the treatment and/or prevention of myopia, preferably in a paediatric population.

## Description

### Technical field

The present invention relates to a preservative free ophthalmic pharmaceutical composition comprising atropine or a pharmaceutically acceptable salt thereof as active ingredient.

### State of the art

Myopia (or short-sightedness) is an ophthalmic condition that leads to blurred long-distance vision, generally characterized by a refractive error of -0.5 or -1 diopters. Overall, it has been estimated that currently 1.4 billion people in the world are myopic (22.9% of the population), and crude estimations suggest that, by 2050, there will be 4.7 billion people affected (nearly 50% of the world population) (Ophthalmology 2016, 123, 1036-1042). Pharmacological treatments have however shown some very promising potential. Of these, mydriatic agents such as atropine or tropicamide gained interest (Asia Pac. J. Ophthalmol. 2018, 7, 405-414), with atropine being the most studied. Its biological action has been surmised as involving a complex interplay with receptors on different ocular tissues at multiple levels, leading to a decrease in change in the cycloplegic refraction and axial length elongation (Pharmaceutics 2020, 12, 781).

Several trials have been performed to prove safety and effectiveness at different concentrations of atropine in controlling myopia progression. Initially tested at 1% concentration during the ATOM1 trial, atropine eyedrops were proven to effectively control myopic progression but caused important visual side effects resulting from cycloplegia and mydriasis (Ophthalmology 2006, 113, 2285-2291). Since then, several clinical trials have evaluated the safety and efficiency of atropine eye drops at lower concentrations. The ATOM2 trial studied myopia progression in 400 children 2 years after treatment with 0.01%, 0.1%, and 0.5% and found the 0.01% concentration to be the concentration causing the least side effects with comparable efficacy in controlling myopia progression (Ophthalmology 2012, 119, 347-354; Ophthalmology 2016, 123, 391-399). Very recently, the LAMP phase 2 trial report confirmed that concentrations ranging from 0.01% to 0.05% were well tolerated in 383 children after two years of treatment, with patients experiencing rare and mild side effects (Ophthalmology 2019, 126, 113-124), even if the need for photochromatic glasses was higher than 30% for treated patients.

Despite all this recent and very favourable clinical data, there is still currently no commercially available low dose atropine ophthalmic compositions.

The catabolic degradation of aqueous atropine is well described. Instability is due to either hydrolysis, which yields tropine and tropic acid, or dehydration, which yields apoatropine. The stability of aqueous atropine sulfate is dependent on several variables. It is well established that stability is enhanced in acid solution. Ideal storage pH for aqueous atropine sulfate solution ranges approximately between 3 and 4 (Lund W, Waaler T. The kinetics of atropine and apoatropine in aqueous solutions. Acta Chem Scand. 1968; 22:3085―97). However, eye pain and stinging may occur upon the instillation of acidic atropine sulfate ophthalmic solution. This could jeopardize the compliance of a long treatment as it is the case for the treatment of myopia in children. Therefore, atropine sulfate ophthalmic solutions with a more acidic pH need to be formulated to allow the eye to adjust the pH to a physiological value of 7.4 as quick as possible.

The international patent application WO2017204262-A2 discloses aqueous composition comprising 0.001 - 0.1 % (w/v) atropine or a salt thereof, a buffer (I) and a water-soluble polymer, which is at a pH range of 6 or lower. It is described that the buffer (I) is at least one selected from the group consisting of a phosphate buffer, an aminocarboxylate buffer, a carbonate buffer, an acetate buffer, a tartrate buffer, a borate buffer, and trometamol. Preferred buffer (I) is phosphate buffer. This aqueous composition further comprises a buffer (II), preferably citrate buffer. It is stated that this aqueous composition has a potent action for inhibiting the elongation of eye axial length and improving the refractive error without exacerbating the mydriatic action of atropine. Additionally, a non-ionic tonicity agent is needed to inhibit the debasement over time of the viscosity given by the water-soluble polymer and additionally maintain the stability of atropine or a salt thereof. The stability of atropine was studied by measuring the amounts of tropic acid. The study showed an increase in tropic acid in only four weeks, and this is less than desirable.

The international patent application WO2015200361-A1 disclosed ophthalmic compositions comprising from about 0.001 wt% to about 0.05 wt% of atropine or atropine sulfate, water and a buffering agent to provide a pH from about 3.8 to about 7.5. Several ophthalmic water solutions are described in the examples comprising either citric acid or acetic acid. Compositions comprising citric acid are described to have a pH 5.8, 6.4 and 6.8. Compositions comprising acetic acid are described to have a pH of 4.2 and 4.8. The stability of these low concentration ophthalmic compositions of atropine is addressed in relation to the use of deuterated water. It is disclosed that deuterated water has lowered buffering capacity than H2O. Therefore, ophthalmic formulations or solutions formulated in deuterated water allow them to reach physiological pH when administered into the eye at a faster rate than compared to an equivalent ophthalmic formulation or solution formulated in H₂O.

The international patent application WO2018209051-A1 discloses liquid storage-stable low-dose ophthalmic compositions comprising equal or less than 0.05 wt% atropine or a pharmaceutically acceptable salts thereof, a buffer, a tonicity agent and a viscosity modifier. These compositions are described to be stable with a pH near to a neutral pH between 5.0 and 6.0 and using buffering systems with a concentration from about 10 mM to about 75mM. These compositions are free of preservative. Several viscosity modifiers are cited, and cellulosic viscosity modifiers are said to be preferred.
There it is disclosed that the stability of the atropine in these compositions is bounded by the use of a specific low concentration of buffering system at also a specified pH range in combination with the other cited components, such as the presence of a viscosity modifier. Specifically, it is disclosed that once the buffer concentration was adjusted to 10 mM-75 mM at a pH of between 5.0-6.0, the stability of the atropine increases with less formation of tropic acid (a byproduct of atropine hydrolysis). Several examples are disclosed in WO2018209051-A1 in tables 1-3. All these examples disclose aqueous atropine sulfate compositions with a viscosity modifier, specifically hypromellose 2910. The buffer cited in the examples is always monobasic and dibasic sodium phosphate buffer.

The international patent application WO2020219707-A1 discloses topical ophthalmic compositions comprising a list of active components wherein atropine is among them and further a buffer, having a pH of about 3.0 to about 5.5 and additionally, these compositions do not contain a viscosity-enhancing component. The concentration of atropine is described from 0.01 to 2% w/w. A suitable buffer is described to stabilize the stored compositions by maintaining the compositions at a low pH (e.g., pH of about 3.0 to 7.4, or about 3.0 to about 6.0, or about 3.0 to about 5.5), but that quickly equilibrates to a physiological pH (i.e., the pH of the tear film and/or ocular surface, or a pH of about 7.0) when the compositions are administered to the surface of an eye. These topical ophthalmic compositions may contain any one of the buffers listed in table 2: boric acid buffer, borate buffer, borate citrate buffer and lactate buffer. Optionally these compositions also comprise a secondary buffering agent that includes citrate buffer and acetate buffer (Table 3). These compositions can further comprise a preservative.

Several examples are disclosed in WO2020219707-A1. Example 2 discloses Formulations 1-4 comprising 1% w/w of atropine sulfate with boric acid as buffer and sodium citrate dihydrate as a secondary buffering agent (Formulation 1-2) or with sodium lactate as buffer and sodium citrate dihydrate as a secondary buffering agent (Formulation 3-4). All Formulations 1-4 were adjusted to pH 6.0. Example 4 discloses 1% atropine aqueous formulation (Formulation C). Boric acid is the buffer used and further sodium citrate dihydrate as a secondary buffering agent. The pH is adjusted to 4.35. The stability of this formulation is not disclosed. Example 5 discloses 3 formulations (Formulation I-III) with 0.03% w/w of atropine sulfate and also having boric acid and sodium citrate dihydrate as a mixture of buffers. These formulations were described to have an initial (T₀) pH of 4.74, 4.80 and 4.79, respectively. In all the cases, the pH was not stable and gradually decreased along the time, reaching 4.45. 4.56 and 4.31 after 6 months at 25°C/40%RH, respectively. The stability study results suggest that the mixture of both buffers, along with the presence of glycine or benzalkonium chloride do not provide sufficient stability for 0.03% atropine aqueous formulations.

The international patent application WO9526711 describes ophthalmic composition in the form of topical solutions consisting of an ophthalmologically active agent, an ion sensitive, a hydrophilic polymer in an amount of 0.004 to 1.5 % by weight, at least one salt selected from the group of inorganic salts and buffers in a total amount from 0.01 to 2.0 % by weight. Optionally, these compositions can also contain a wetting agent, a preservative and a pH regulating agent. It is described that these solutions exhibit a pH of 4.0 to 8.0, preferably 6.5 to 8.0 and a viscosity of less than 1000 mPas. Several typical examples of basic drug molecules useful in eye therapy are described with atropine being one of them. Timolol or salt thereof is described as preferred, and all the examples are related to timolol. The stability of these eye drops is not addressed.

Despite the ophthalmic solutions available so far in the art, there is still the need to provide further ophthalmic formulations comprising low concentration of atropine sulfate which remain stable, i.e., substantially resistant against hydrolysis, with an assay of atropine within an acceptable range and free of degradation products, over time and are suitable for use for the treatment of myopia, including also paediatric population. Furthermore, these ophthalmic formulations are easy to be manufactured through a low cost and automatized process.

### Object of the invention

The object of the present invention is preservative free ophthalmic low concentration atropine sulfate composition containing citrate buffer.

Another aspect of the invention is a pharmaceutical dosage form comprising said ophthalmic composition.

Another aspect of the invention is a process for the preparation of such ophthalmic composition.

Another aspect of the invention is the preservative free ophthalmic low concentration atropine sulfate composition for use in the treatment for use in therapy, preferably, for the treatment and/or prevention of myopia, preferably in a paediatric population.

### Detailed description of the invention

The object of the present invention is a preservative free ophthalmic low concentration atropine sulfate composition consisting of:
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH from about 4.0 to about 5.5.

The authors of the present invention have developed a preservative free ophthalmic low concentration atropine sulfate composition consisting of atropine sulfate, citrate buffer in an amount of less than about 100mM, preferably about 10mM, a tonicity agent and water wherein the required stability of the atropine in ophthalmic compositions is achieved as well as the comfort of the eye upon the instillation of the drop in the eye is guaranteed by adjusting the pH of the solution from 4.0 to 5.5.

Therefore, according to the present invention, the use of citrate buffer (with pKa of 4.76) to adjust the solution to the desired pH from about 4.0 to about 5.5 allows to use significantly less concentration of the buffer present in the ophthalmic composition and without the addition of any additional buffer. This is due to the buffer capacity depends on the concentration of the buffer components in the buffered solution, and it rises to a maximum at pH=pKₐ. Another benefit of using citrate buffer as a sole buffer is that the side effects that other buffers can cause in the eye, such as the corneal calcification associated with the use of eye drops containing phosphate buffer, are totally avoided. Additionally, the use of a significantly lower amount of citrate buffer guarantees the comfort of the eye upon instillation of an ophthalmic composition having a pH from about 4.0 to about 5.5. This less buffer concentrated ophthalmic composition makes the eye adjust easier and faster the pH of the inserted drop to the physiological value since the ophthalmic composition gives less resistance to pH changes/alterations.

Further, prior art compositions contain higher buffer amount, for example with anhydrous phosphate buffer components in WO2018209051, where decrease in buffer concentration from 100mM to 50mM significantly increase the pH value fluctuation. Surprisingly, the authors of the present invention have found that the use of citrate buffer allows to design a stable low dose atropine sulfate formulations, in which the assay of atropine as well as the total level of impurities are within a tolerance limit for the ophthalmic compositions. These benefits are observed, when essentially a small amount of the buffering agent is used. The application of the citrate buffer also minimalizes the irritating effect of the formulation and guarantees the comfort of the eye upon the installation of the drop in the eye. Moreover, the presence of citrate buffer enables precise control of the pH at the desired range using regular and constant amounts of each citrate buffer components. This is notoriously observed when each citrate buffer component is both hydrate, such as sodium citrate dihydrate and citric acid monohydrate. The precise control of the pH at the desired ranges, ensure the stability of atropine in the compositions of the present invention. Additionally, surprisingly the authors of the present invention have found that the use of citrate buffer as buffer ensures the control of the pH to the extent that no pH adjuster is needed. This is the case within a specific range of molar ratio between citric acid and sodium citrate. This range is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

Along the present description, as well as in the claims, the singular expressions, generally preceded by the articles "a", "an" or "the", are meant to include also the plural forms, unless the context clearly indicates otherwise. Furthermore, if it is not stated on the contrary, numeric values preceded by the term "about" are meant to include the exact stated value and also a certain variation around such value, namely a variation of ±5% of the stated amount. Numeric ranges defined by lower and upper endpoints are meant to include also said stated endpoints. The percentages disclosed are as "w/v%" meaning weight by volume percentages or as w/w% meaning weight by weight percentages. Moreover, it is noted that weight percentages of atropine sulfate provided herein are based on atropine sulfate monohydrate.

### Atropine

Atropine occurs naturally in a number of plants of the nightshade family, including deadly nightshade, Jimson weed, and mandrake. It was first isolated in 1833 (Neurological aspects of substance abuse (2 ed.), 309-315 (2004). It is on the World Health Organization's List of Essential Medicines, the most effective and safe medicines needed in a health system. The chemical structure of atropine is shown in Figure I.

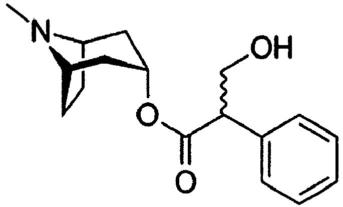

Atropine is currently the most effective therapy for myopia control. Recent clinical trials demonstrated low-dose atropine eye drops such as 0.01% resulted in retardation of myopia progression, with significantly less side effects compared to higher concentration preparation.

The exact mechanism of topical atropine is still not known, although the up- and downregulation of retinal and scleral muscarinic receptors with influence on the scleral matrix have been postulated. Moreover, atropine inhibits myopia induction in both mammalian and avian eyes. Different to the mammalian eye, the avian eye contains striated ciliary muscle innervated by nicotinic receptor rather than muscarinic receptors. Therefore, atropine might have a function at a relatively lower dose, through M1/M4 receptors in the retina, not via the accommodation system. On the other hand, a non-muscarinic and a direct influence of atropine on the scleral fibroblasts could also contribute to the effect [Pei-Chang Wu, Meng-Ni Chuang, Update in myopia and treatment strategy of atropine use in myopia control, Eye (2019) 33:3―13].

Despite all this recent and very favourable clinical data, there is still currently no commercially available low dose atropine ophthalmic compositions.

### Ophthalmic composition

The preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH from about 4.0 to about 5.5.

The amount of the atropine sulfate in the preservative free ophthalmic low concentration atropine sulfate composition according to the present invention is from about 0.005 w/w% to about 0.05 w/w%. Generally, the amount of atropine sulfate in the composition is from about 0.006 w/w% to about 0.05 w/w%, preferably is from about 0.007 w/w% to about 0.05 w/w%, preferably from about 0.008 w/w% to about 0.05 w/w%, preferably from about 0.009 w/w% to about 0.05 w/w%, more preferably from about 0.01 w/w% to about 0.05 w/w%. Still more preferably the amount of atropine sulfate in the composition is 0.01 w/w% or 0.05 w/w%. In the preservative free ophthalmic low concentration atropine sulfate composition according to the present invention the concentration of the atropine sulfate as w/w% is equivalent to w/v%.

The preservative free ophthalmic low concentration atropine sulfate composition has a pH from about 4.0 to about 5.5, preferably from about 4.0 to about 5.4, preferably from about 4.0 to about 5.3, preferably from about 4.0 to about 5.2, preferably from about 4.0 to about 5.1, preferably from about 4.0 to about 5.0, preferably from about 4.0 to about 4.9, preferably from about 4.0 to about 4.8, preferably from about 4.0 to about 4.7, preferably from about 4.0 to about 4.6, preferably from about 4.0 to about 4.5, preferably from about 4.0 to about 4.4, preferably from about 4.0 to about 4.3, preferably from about 4.0 to about 4.2, preferably from about 4.0 to about 4.1, preferably from about 4.1 to about 5.5, preferably from about 4.1 to about 5.4, preferably from about 4.1 to about 5.3, preferably from about 4.1 to about 5.2, preferably from about 4.1 to about 5.1, preferably from about 4.1 to about 5.0, preferably from about 4.1 to about 4.9, preferably from about 4.1 to about 4.8, preferably from about 4.1 to about 4.7, preferably from about 4.1 to about 4.6, preferably from about 4.1 to about 4.5, preferably from about 4.1 to about 4.4, preferably from about 4.1 to about 4.3, preferably from about 4.1 to about 4.2, preferably from about 4.2 to about 5.5, preferably from about 4.2 to about 5.4, preferably from about 4.2 to about 5.3, preferably from about 4.2 to about 5.2, preferably from about 4.2 to about 5.1, preferably from about 4.2 to about 5.0, preferably from about 4.2 to about 4.9, preferably from about 4.2 to about 4.8, preferably from about 4.2 to about 4.7, preferably from about 4.2 to about 4.6, preferably from about 4.2 to about 4.5, preferably from about 4.2 to about 4.4, preferably from about 4.2 to about 4.3, preferably from about 4.3 to about 5.5, preferably from about 4.3 to about 5.4, preferably from about 4.3 to about 5.3, preferably from about 4.3 to about 5.2, preferably from about 4.3 to about 5.1, preferably from about 4.3 to about 5.0, preferably from about 4.3 to about 4.9, preferably from about 4.3 to about 4.8, preferably from about 4.3 to about 4.7, preferably from about 4.3 to about 4.6, preferably from about 4.3 to about 4.5, preferably from about 4.3 to about 4.4, preferably from about 4.4 to about 5.5, preferably from about 4.4 to about 5.4, preferably from about 4.4 to about 5.3, preferably from about 4.4 to about 5.2, preferably from about 4.4 to about 5.1, preferably from about 4.4 to about 5.0, preferably from about 4.4 to about 4.9, preferably from about 4.4 to about 4.8, preferably from about 4.4 to about 4.7, preferably from about 4.4 to about 4.6, preferably from about 4.4 to about 4.5, preferably from about 4.5 to about 5.5, preferably from about 4.5 to about 5.4, preferably from about 4.5 to about 5.3, preferably from about 4.5 to about 5.2, preferably from about 4.5 to about 5.1, preferably from about 4.5 to about 5.0, preferably from about 4.5 to about 4.9, preferably from about 4.5 to about 4.8, preferably from about 4.5 to about 4.7, preferably from about 4.5 to about 4.6, more preferably is about 4.5. Still more preferably the preservative free ophthalmic low concentration atropine sulfate composition has a pH from about 4.0 to about 5.0, preferably from about 4.0 to about 4.5, more preferably about 4.5. In some embodiments the composition has a pH from about 4.0 to about 4.4, preferably from about 4.2 to about 4.4. In specific pH case, the term "about" means to include the exact stated pH value and also a certain variation around such pH value, namely a variation of ±1% of the stated pH value.

The preservative free ophthalmic low concentration atropine sulfate composition according to the present invention may also contain a buffer. The term "buffer" refers to a substance capable in solution of neutralizing both acids and bases and thereby maintaining the original acidity or basicity of the solution. Specific examples of buffers include but are not limited to boric acid, borax, citric acid, disodium hydrogenphosphate, ε-aminocaproic acid and the like. The buffer suitable for the ophthalmic composition of the present invention is citrate buffer. The citrate buffer includes citric acid and sodium citrate or any of their hydrate forms. Preferably, the citrate buffer is citric acid monohydrate and sodium citrate dihydrate or any other combinations in which one of the buffer components is in its hydrate form. The concentration of the citrate buffer present in the preservative free ophthalmic low concentration atropine sulfate composition according to the present invention is less than about 100 mM, preferably less than 95 mM, preferably less than 90, preferably less than 85, preferably less than 80 mM, preferably less than 75, preferably less than 70, preferably less than 65 mM, preferably less than 60, preferably less than 55 mM, preferably less than 50 mM, preferably less than 45, preferably less than 40 mM, preferably less than 35, preferably less than 30 mM, preferably less than 25, preferably less than 20 mM, preferably less than 15, and still more preferably 10 mM or less. Still more preferably the amount of the citrate buffer is about 1 mM, about 2 mM, is about 3 mM, about 4 mM, about 5 mM, about 6 mM, is about 7 mM, about 8 mM, about 9 mM, about 10 mM. Still more preferably, the amount of the citrate buffer is about 10 mM, and still more preferably is 10 mM.

The preservative free ophthalmic low concentration atropine sulfate composition according to the present invention also contains a tonicity agent. The term "tonicity agent" refers to a compound designed to reduce local irritation by preventing osmotic shock at the site of application. They are also known as osmotic pressure agents. Suitable tonicity agents include sodium chloride, potassium chloride, calcium chloride, glycerin, propylene glycol, glycerol, sorbitol, mannitol and the like. A preferred tonicity agent is sodium chloride. The amount of the tonicity agent in the preservative free ophthalmic low concentration atropine sulfate composition according to the present invention is from about 0.1 w/v% to about 2.0 w/v%. Preferably from about 0.5 w/v% to 1.0 w/v%, more preferably about 1.0 w/v% and more preferably about 0.87w/v%.

The preservative free ophthalmic low concentration atropine sulfate composition according to the present invention may optionally have a pH adjuster. The preservative free ophthalmic low concentration atropine sulfate composition according to the present invention, i.e. the composition with citrate buffer, normally no pH adjuster is needed. The term "pH adjuster" refers to a chemical compound which is used to alter pH of the aqueous solution within the range from about 4.0 to 5.5. As cited above, his is the case when the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82. Probably and without being bounded to any specific theory, due to fact that the pKa of the citrate buffer is very close to the desired working range of the buffer in the ophthalmic composition of the present invention, i.e, pH from about 4.0 to about 5.5. The use of this molar ratio in the preservative free ophthalmic low concentration atropine sulfate composition according to the present invention ensures the stability of the atropine sulfate in solution. In case that pH has to be adjusted, when the pH needs to be lowered, the pH adjusting agent will be an inorganic acid such as hydrochloric acid or sulfuric acid. And when the pH needs to be increased, the pH adjusting agent will be an inorganic base such as sodium hydroxide or potassium hydroxide. Preferred inorganic acid as pH adjuster is hydrochloric acid. Preferred inorganic base as pH adjuster is sodium hydroxide. The amount of the pH adjuster in the ophthalmic composition is significantly low, less than 0.001 w/v%, preferably less than 0.0001 w/v%, more preferably less than 0.00001 w/v%, more preferably less than 0.000001 w/v%, more preferably less than 0.0000001 w/v%.

The preservative free ophthalmic low concentration atropine sulfate composition according to the present invention also contains water.

As used herein, the term "ophthalmic composition" refers to liquid compositions that are suitable for topical ophthalmic delivery or eye drops, including solutions, suspensions and emulsions. In a specific embodiment the term "ophthalmic composition" refers to an ophthalmic solution. In a specific embodiment the term "ophthalmic composition" refers to an ophthalmic aqueous solution.

Within this description, the "preservative-free ophthalmic solution" means that the ophthalmic solution of the present invention does not comprise a preservative, such as quaternary ammonium salts, e.g. benzalkonium chloride (BAK). Other pharmaceutically acceptable preservatives for ophthalmic solutions are for example boric acid-polyol-zinc chloride or chlorine oxide compounds, chlorhexidine gluconate, benzethonium chloride, sorbic acid, potassium sorbate, ethyl p-hydroxybenzoate and butyl p-hydroxybenzoate.

In a preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH from about 4.0 to about 5.5.

In another preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH from about 4.0 to about 5.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
i) atropine sulfate in an amount from about 0.005 w/w% to about 0.05 w/w%
j) citrate buffer in an amount of less than about 100mM, preferably about 10mM
k) a tonicity agent
I) water
m) optionally a pH adjuster
n) optionally a chelating agent
o) optionally a complexing agent, and
p) optionally a viscosity agent
characterized in that it has a pH from about 4.0 to about 5.0.

In another preferred embodiment, the preservative free ophthalmic low concentration atropine sulfate composition of the present invention is consisting of
i) atropine sulfate in an amount from about 0.005 w/w% to about 0.05 w/w%
j) citrate buffer in an amount of less than about 100mM, preferably about 10mM
k) a tonicity agent
I) water
m) optionally a pH adjuster
n) optionally a chelating agent
o) optionally a complexing agent, and
p) optionally a viscosity agent
characterized in that it has a pH from about 4.0 to about 5.0 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
q) atropine sulfate in an amount from about 0.005 w/w% to about 0.05 w/w%
r) citrate buffer in an amount of less than about 100mM, preferably about 10mM
s) a tonicity agent
t) water
u) optionally a pH adjuster
v) optionally a chelating agent
w) optionally a complexing agent, and
x) optionally a viscosity agent
characterized in that it has a pH from about 4.0 to about 4.5.

In another preferred embodiment, the preservative free ophthalmic low concentration atropine sulfate composition of the present invention is consisting of
q) atropine sulfate in an amount from about 0.005 w/w% to about 0.05 w/w%
r) citrate buffer in an amount of less than about 100mM, preferably about 10mM
s) a tonicity agent
t) water
u) optionally a pH adjuster
v) optionally a chelating agent
w) optionally a complexing agent, and
x) optionally a viscosity agent
characterized in that it has a pH from about 4.0 to about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH about 4.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH about 4.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) a tonicity agent
d) water
e) optionally a pH adjuster
f) optionally a chelating agent
g) optionally a complexing agent, and
h) optionally a viscosity agent
characterized in that it has a pH about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent, and
d) water
characterized in that it has a pH from about 4.0 to about 5.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent, and
d) water
characterized in that it has a pH from about 4.0 to about 5.0.

In another preferred embodiment, the preservative free ophthalmic low concentration atropine sulfate composition of the present invention is consisting of
a) atropine sulfate in an amount of about 0.01w/w% or about 0.05 w/w%
b) citrate buffer in an amount of less than about 100mM, preferably about 10mM
c) sodium chloride as tonicity agent, and
d) water
characterized in that it has a pH about 4.5.

The preservative free ophthalmic low concentration atropine sulfate composition according to the present invention may optionally have a chelating agent. As it is well-known in the field of pharmaceutical technology, chelating agents are commonly used to complex metal ions that can catalyze drug degradation They are also called chelants, chelators or sequestering agents. Suitable chelating agent to be used in this invention include, but are not limited to, ethylenediamine tetraacetic acid (EDTA), disodium edetate, calcium disodium edetate, trisodium edetate, tetrasodium edetate and/or combinations thereof. Preferably, the chelating agent is EDTA as a disodium salt. The amount of the chelating agent in the ophthalmic composition is generally from about 0.01% w/w to about 0.1% w/w, preferably 0.01% w/w. Surprisingly, the authors of the present invention have found that the presence of a chelating agent, preferably EDTA, in the preservative free ophthalmic low concentration atropine sulfate composition of the present invention increases the stability of atropine sulfate, and hence decreases the concentration of impurities, which are responsible for both fluctuations of the pH value as well as irritating effect of the composition. Moreover, EDTA substantially increases the corneal permeability of atropine, thus improving the effectiveness of the drug.

In a preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent, preferably EDTA
g) optionally a complexing agent, and
h) optionally a viscosity agent
characterized in that it has a pH from about 4.0 to about 5.5.

In a preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent, preferably EDTA
g) optionally a complexing agent, and
h) optionally a viscosity agent
characterized in that it has a pH from about 4.0 to about 5.0.

In another preferred embodiment, the preservative free ophthalmic low concentration atropine sulfate composition of the present invention is consisting of
a) atropine sulfate in an amount from about 0.005 w/w% to about 0.05 w/w%
b) citrate buffer in an amount of less than about 100mM, preferably about 10mM
c) a tonicity agent
d) water
e) optionally a pH adjuster
f) optionally a chelating agent, preferably EDTA
g) optionally a complexing agent, and
h) optionally a viscosity agent
characterized in that it has a pH from about 4.0 to about 5.0 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) optionally a chelating agent, preferably EDTA
characterized in that it has a pH from about 4.0 to about 5.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) optionally a chelating agent, preferably EDTA
characterized in that it has a pH from about 4.0 to about 5.0.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) optionally a chelating agent, preferably EDTA
characterized in that it has a pH about 4.5.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) optionally a chelating agent, preferably EDTA
characterized in that it has a pH about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) a chelating agent, preferably EDTA
characterized in that it has a pH about 4.5.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) a chelating agent, preferably EDTA
characterized in that it has a pH about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

The preservative free ophthalmic low concentration atropine sulfate composition according to the present invention may optionally have a complexing agent. Complexing agents are compounds, which can form relatively stable complexes with drugs by somewhat strong coordinate covalent bonds or by relatively weak noncovalent forces such as hydrogen bonds, van der Waals forces, electrostatic interactions, dipole forces or hydrophobic interactions. In the ophthalmic products complexing agent are mainly used to increase the aqueous solubility of poorly water-soluble drugs, to increase their bioavailability and stability as well as reduce the ocular irritation or prevent drug-drug or drug-additive interactions. Said complexing agents are typically cyclodextrins, as are well for the skilled person in pharmaceutical composition. Suitable cyclodextrins to be used in the compositions of this invention are β-cyclodextrin, γ- cyclodextrin, hydroxypropyl-β-cyclodextrin, sulfobutylether β-cyclodextrin. Still more preferably, the complexing agent is hydroxypropyl-β-cyclodextrin. The amount of the complexing agent in the ophthalmic composition is generally from about 0.001% w/w to about 1% w/w, preferably from about 0.005% w/w to about 0.2% w/w, more preferably from about 0.01% w/w to about 0.1% w/w, more preferably from about 0.015% w/w to about 0.05% w/w, more preferably from about 0.02% w/w to about 0.025% w/w,and more preferably about 0.022% w/w. It has been found that the further presence of complexing agent allows to form an inclusion complex with atropine molecule, which increases its long-term stability.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin
characterized in that it has a pH from about 4.0 to about 5.5.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin
characterized in that it has a pH from about 4.0 to about 5.0.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-p-cyclodextrin
characterized in that it has a pH from about 4.0 to about 5.0 and and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin
characterized in that it has a pH from about 4.0 to about 5.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-p-cyclodextrin
characterized in that it has a pH from about 4.0 to about 5.0.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin
characterized in that it has a pH about 4.5.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin characterized in that it has a pH about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid, and
f) a complexing agent, preferably hydroxypropyl-β-cyclodextrin
characterized in that it has a pH about 4.5.

The preservative free ophthalmic low concentration atropine sulfate composition according to the present invention may optionally have a viscosity agent. A viscosity agent is a compound which significantly increase the viscosity of the formulation. In ocular drug delivery systems, a viscosity agent prolongs corneal contact time and, thus, improves drug bioavailability. Said viscosity agents are typically pharmaceutically acceptable polymers, as are well known for the skilled person in pharmaceutical formulation. Suitable pharmaceutically acceptable polymers include, for example, microcrystalline cellulose, hydroxypropyl methylcellulose (Hypromellose, HPMC), hydroxyethyl cellulose (HEC), carboxy methylcellulose, polyvinyl alcohol (PVA), polyethylene glycol (PEG) or natural polymers, such as hyaluronic acid, guar gum, gellan gum and the mixture thereof. Preferably, the pharmaceutically acceptable polymers are hydroxypropyl methylcellulose and hydroxyethyl cellulose. More preferably, hydroxyethyl cellulose. The amount of the pharmaceutically polymer in the ophthalmic composition is generally from about 0.01 to about 0,5% w/v, preferably from about 0.1% w/v to about 0,35% w/v, more preferably from about 0,15% w/v to about 0.25% w/v.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) water
d) optionally a pH adjuster, preferably hydrochloric acid
e) optionally a chelating agent, preferably EDTA
f) optionally a complexing agent, preferably hydroxypropyl-β-cyclodextrin
g) optionally a viscosity agent, preferably hydroxyethyl cellulose
characterized in that it has a pH from about 4.0 to about 5.5.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) water
d) optionally a pH adjuster, preferably hydrochloric acid
e) optionally a chelating agent, preferably EDTA
f) optionally a complexing agent, preferably hydroxypropyl-β-cyclodextrin
g) optionally a viscosity agent, preferably hydroxyethyl cellulose
characterized in that it has a pH from about 4.0 to about 5.0.

In a preferred embodiment, the preservative free ophthalmic low concentration atropine sulfate composition of the present invention is consisting of
a) atropine sulfate in an amount of about 0.005 w/w% to about 0.05 w/w%
b) citrate buffer in an amount of less than about 100mM, preferably about 10mM
c) water
d) optionally a pH adjuster, preferably hydrochloric acid
e) optionally a chelating agent, preferably EDTA
f) optionally a complexing agent, preferably hydroxypropyl-β-cyclodextrin
g) optionally a viscosity agent, preferably hydroxyethyl cellulose characterized in that it has a pH from about 4.0 to about 5.0 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) water
d) optionally a pH adjuster, preferably hydrochloric acid
e) optionally a chelating agent, preferably EDTA
f) optionally a complexing agent, preferably hydroxypropyl-β-cyclodextrin
g) optionally a viscosity agent, preferably hydroxyethyl cellulose
characterized in that it has a pH from about 4.0 to about 5.5.

In a preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) water
d) optionally a pH adjuster, preferably hydrochloric acid
e) optionally a chelating agent, preferably EDTA
f) optionally a complexing agent, preferably hydroxypropyl-β-cyclodextrin
g) optionally a viscosity agent, preferably hydroxyethyl cellulose
characterized in that it has a pH from about 4.0 to about 5.0.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-p-cyclodextrin, and
h) optionally a viscosity agent, preferably hydroxyethyl cellulose
characterized in that it has a pH about 4.5.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-β-cyclodextrin, and
h) optionally a viscosity agent, preferably hydroxyethyl cellulose characterized in that it has a pH about 4.5 and the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

In a more preferred embodiment, the preservative free ophthalmic low concentration atropine sulfate composition of the present invention is consisting of
a) atropine sulfate in an amount of about 0.01 w/w% or about 0.05 w/w%
b) citrate buffer in an amount of less than about 100mM, preferably about 10mM
c) sodium chloride as tonicity agent
d) water
e) optionally a pH adjuster, preferably hydrochloric acid
f) optionally a chelating agent, preferably EDTA
g) optionally a complexing agent, preferably hydroxypropyl-P-cyclodextrin, and
h) a viscosity agent, preferably hydroxyethyl cellulose
characterized in that it has a pH about 4.5.

### Dosage form

The pharmaceutical dosage form of the present invention is an ophthalmic composition is the form of an aqueous solution.

Typically, the aqueous solution is filled container. A container is referred herein also as a bottle, reservoir or vessel. Typically, containers for ophthalmic compositions are made of a polymer. Suitable polymers include, but are not limited to, polyethylene (PE), polypropylene (PP), polyethylene terephthalate (PET), polyvinyl chloride, acrylic resins, polystyrene, polymethylmethacrylate, nylon 6 and mixtures thereof. In a preferred embodiment, the container is made of polyethylene (PE) or polypropylene (PP). These polymers can be high-density or low-density polymers, e.g high density polyethylene.

Containers made of PP as well as PE are shatterproof and lightweight. These materials are well known and used for decades. In relation to the extractables they are considered safe and neutral to the packaging content.

Said containers for the aqueous solution can be manufactured by both an injection-blow moulding and a blow-fill-seal processes, which are widely known in pharmaceuticals industry. In a preferred embodiment, the container is manufactured by the injection-blow moulding process.

Typically, the containers suitable for ophthalmic compositions have a dropper system. The preservative free ophthalmic low concentration atropine sulfate composition of the present invention refers to a multidose use of the composition. Typically, ophthalmic compositions suitable for multidose use have to guarantee the sterility of the solution after first opening of the device and during following uses. Preservatives are commonly added to guarantee this sterility. However, they can be toxic and pose problems of tolerance, especially in the context of a long-term treatment such as myopia. In preservative free ophthalmic compositions of the present invention, sterility is guaranteed with specific dropper systems, which are tightly attached to the container. Suitable dropper systems for a multidose use of the preservative free ophthalmic solutions may have a special pump or require a bottle squeezing to open a valve and deliver the drop with an exact volume. In a preferred embodiment, the dropper system is equipped with the pump.

### Preparation of the dosage form

Another aspect of the present invention is a process for the preparation of the preservative free ophthalmic low concentration atropine sulfate composition, as defined above, comprising the following steps:
i) dissolve all the components except atropine sulfate in water
ii) dissolve the atropine sulfate in the solution obtained in step i)
iii) sterile filtration of the solution obtained in step ii)
iv) dispense the filtered solution obtained in step iii) to a sterile container
v) aseptic closure of the container obtained in step iv) by a sterile dropper or dropper system suitable for ophthalmic compositions

### Use of the dosage form

As shown in the stability test disclosed in Examples 1A and 1B and 2B, 2C and 2D, surprisingly, the dosage form according to the present invention is outstandingly stable and is therefore particularly suitable for myopia.

Furthermore, as shown in Examples 1A and 1B and 2C and 2D, the dosage form of the invention has particularly low levels of tropic acid impurities.

Then, the present dosage form has advantages over the prior art solutions due to the reduced amount of buffer used in the composition, particularly, the lower amount of citrate buffer. Therefore, the present dosage form comprising the ophthalmic composition of the present invention is advantageous in terms of safety, as the possible adverse events related to some buffers used for ophthalmic solutions are minimized.

Therefore, another aspect of the invention is the preservative free ophthalmic low concentration atropine sulfate composition according to the present invention, or the pharmaceutical dosage form comprising it, for use in therapy, preferably, for use in the treatment and/or prevention of myopia, preferably in a paediatric population.

Another aspect of the present invention is a method for treating and/or preventing myopia in a patient in need thereof, preferably in a paediatric population, comprising the step of administering therapeutically effective amount of the preservative free ophthalmic low concentration atropine sulfate composition or the dosage form of the present invention.

The present invention relates to the following embodiments:
A preservative free ophthalmic low concentration atropine sulfate composition consisting of
   a) atropine sulfate in an amount of about 0.01 w/w% or about 0.05 w/w%
   b) citrate buffer in an amount of less than about 100mM, preferably about 10 mM
   c) sodium chloride as tonicity agent
   d) water, and
   e) optionally a chelating agent, preferably EDTA
   characterized in that it has a pH from about 4.0 to about 5.5.

A preservative free ophthalmic low concentration atropine sulfate composition consisting of
f) atropine sulfate in an amount of about 0.01 w/w% or about 0.05 w/w%
g) citrate buffer in an amount of less than about 100mM, preferably about 10 mM
h) sodium chloride as tonicity agent
i) water, and
j) optionally a chelating agent, preferably EDTA
characterized in that it has a pH from about 4.0 to about 5.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) optionally a chelating agent, preferably EDTA
characterized in that it has a pH from about 4.0 to about 5.0.

A preservative free ophthalmic low concentration atropine sulfate composition consisting of
f) atropine sulfate in an amount of about 0.01 w/w% or about 0.05 w/w%
g) citrate buffer in an amount of less than about 100mM, preferably about 10 mM
h) sodium chloride as tonicity agent
i) water, and
j) optionally a chelating agent, preferably EDTA
characterized in that it has a pH from about 4.0 to about 5.0 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) optionally a chelating agent, preferably EDTA
characterized in that it has a pH from about 4.0 to about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) optionally a chelating agent, preferably EDTA
characterized in that it has a pH from about 4.0 to about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) optionally a chelating agent, preferably EDTA
characterized in that it has a pH about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) optionally a chelating agent, preferably EDTA
characterized in that it has a pH about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 5.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 5.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 5.0.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 5.0 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH from about 4.0 to about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent
characterized in that it has a pH about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent, and
f) hydroxypropyl-p-cyclodextrin as complexing agent
characterized in that it has a pH from about 4.0 to about 5.5, preferably from about 4.0 to 5.0, more preferably from 4.0 to about 4.5, still more preferably about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent, and
f) hydroxypropyl-β-cyclodextrin as complexing agent
characterized in that it has a pH from about 4.0 to about 5.5, preferably from about 4.0 to 5.0, more preferably from 4.0 to about 4.5, still more preferably about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent, and
f) hydroxyethyl cellulose as viscosity agent
characterized in that it has a pH from about 4.0 to about 5.5, preferably from about 4.0 to 5.0, more preferably from 4.0 to about 4.5, still more preferably about 4.5.

A preservative free ophthalmic low concentration atropine sulfate composition consisting of
a) atropine sulfate in an amount of about 0.01 w/w% or about 0.05 w/w%
b) citrate buffer in an amount of less than about 100mM, preferably about 10 mM
c) sodium chloride as tonicity agent
d) water, and
e) EDTA as chelating agent, and
f) hydroxyethyl cellulose as viscosity agent
characterized in that it has a pH from about 4.0 to about 5.5, preferably from about 4.0 to 5.0, more preferably from 4.0 to about 4.5, still more preferably about 4.5 and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

A process for the preparation of the preservative free ophthalmic low concentration atropine sulfate composition according to this invention comprising the following steps:
i) dissolve all the components except atropine sulfate in water
ii) dissolve the atropine sulfate in the solution obtained in step i)
iii) sterile filtration of the solution obtained in step ii)
iv) dispense the filtered solution obtained in step iii) to a sterile container
v) aseptic closure of the container obtained in step iv) by a sterile dropper or dropper system suitable for ophthalmic compositions

A preservative free ophthalmic low concentration atropine sulfate composition according to this invention for use in therapy, preferably, for use in the treatment and/or prevention of myopia, preferably in a paediatric population.

### Examples

### Example 1. Ophthalmic preservative free low concentration atropine sulfate composition with citrate buffer

Solutions 1A and 1B comprising 0.01% w/w and 0.05 %w/w of atropine sulfate were prepared using the ingredients disclosed in Table 1.

**TABLE 1**

| **Ingredient** | **Example 1A** | **Example 1B** |
|---|---|---|
| | **% w/w** | **% w/w** |
| Atropine sulfate | 0.01 | 0.05 |
| Sodium chloride | 0.87 | 0.87 |
| Disodium edetate | 0.01 | 0.01 |
| Sodium citrate dihydrate | 0.16 | 0.16 |
| Citrate acid monohydrate | 0.095 | 0.095 |
| Water for injections | up to 1 ml | up to 1 ml |
| pH | 4.5 | 4.5 |

The preservative free ophthalmic low concentration atropine sulfate compositions were prepared by dissolving the excipients in 2/3 water. Then atropine sulfate was added and after complete dissolution the rest of the water was added. pH is checked. No pH adjuster solution was needed to adjust the pH. Next, the sterile filtration through 0.2 microns filter was carried out. The sterile solution is filled in sterilized HPDE bottles, and the bottles are tightly closed by the dropper system with a special pump. Finished dosage form was storage at temperature below 25°C. The pharmaceutical compositions of Table 1 were subjected to accelerated stability studies at 40 °C ± 2 °C / 75% ± 5% RH for 2 months.

| **Example 1A - 40 °C ± 2 °C / 75% ± 5% RH** | | | |
|---|---|---|---|
| | initial | 1 month | 2 months |
| pH | 4.50 | 4.50 | 4.50 |
| osmolarity | 302 | 302 | 303 |
| Assay of atropine sulfate / % of w/w | 102.8 | 104.6 | 99.0 |
| Impurity C (tropic acid) / % of w/v | 0.00 | 0.10 | 0.20 |
| Total impurities / % of w/v | 0.00 | 0.10 | 0.20 |

| **Example 1A - 40 °C ± 2 °C / 75% ± 5% RH** | | | |
|---|---|---|---|
| | initial | 1 month | 2 months |
| pH | 4.50 | 4.50 | 4.50 |
| osmolarity | 303 | 303 | 303 |
| Assay of atropine sulfate / % of w/w | 102.6 | 105.9 | 101.0 |
| Impurity C (tropic acid) / % of w/v | 0.00 | 0.11 | 0.20 |
| Total impurities / % of w/v | 0.00 | 0.11 | 0.20 |

It has been found that the pharmaceutical compositions of Table 1 possess excellent stability at 40 °C ± 2 °C / 75% ± 5% RH. The assay of atropine is within a tolerance limit, and the total level of impurity C is below 0.3% through the study durations. Moreover, the pH value and osmolality of each of Examples 1A and 1B remained relatively unchanged, within acceptable levels in terms of stability.

### Example 2. Impact of the pH on the stability of atropine sulfate formulations

Solutions 2A, 2B, 2C and 2D comprising 0.01% w/w of atropine sulfate were prepared analogously as disclosed in Example 1, using the ingredients of Table 2:

**TABLE 2**

| **Ingredient** | **Example 2A** | **Example 2B** | **Example 2C** | **Example 2D** |
|---|---|---|---|---|
| | **% w/w** | **% w/w** | **% w/w** | **% w/w** |
| Atropine sulfate | 0.01 | 0.01 | 0.01 | 0.01 |
| Sodium chloride | 0.49 | 0.44 | 0.50 | 0.71 |
| Sodium citrate dihydrate | 1.47 | 1.44 | 1.03 | 0.47 |
| Citrate acid monohydrate | 0.03 | 0.31 | 0.6 | 0.29 |
| Disodium edetate | 0.01 | 0.01 | 0.01 | 0.01 |
| Hydrochloric acid | - | - | - | - |
| Water for injections | Up to 1 mL | Up to 1 mL | Up to 1 mL | Up to 1 mL |
| pH | 6.5 | 5.3 | 4.5 | 4.3 |

The pharmaceutical compositions of Table 2 were subjected to accelerated stability studies at 40 °C ± 2 °C / 75% ± 5% RH as per the International Committee on Harmonization stability conditions for 3 months. Lab scale batches of atropine sulfate, with a citrate buffer or a pH adjuster, were manufactured and approximately 5 ml of each solution were distributed into the preservative free PE containers. Next, the containers were tightly closed by the dropper system with a pump and the dispensing devices were stored in inverted position. Samples were measured after 1 month and 2 and 3 months. Only in the case of Example 2A, the studies were shortened to 1 month due to very low stability of the composition. The results are shown in Tables 3-6.

**TABLE 3**

| **Example 2A - 40 °C ± 2 °C / 75% ± 5% RH** | | | | |
|---|---|---|---|---|
| | initial | 1 month | 2 months | 3 months |
| pH | 6.50 | 6.50 | - | - |
| osmolarity | 299 | 299 | - | - |
| Assay of atropine sulfate / % of w/w | 101.80 | 86.0 | - | - |
| Impurity C (tropic acid) / % of w/v | 0 | 0 | - | - |
| Total impurities / % of w/v | 0.13 | 16.86 | - | - |

**TABLE 4**

| **Example 2B - 40 °C ± 2 °C / 75% ± 5% RH** | | | | | |
|---|---|---|---|---|---|
| | initial | 1 month | 2 months | 3 months | 6 months |
| pH | 5.30 | 5.30 | 5.30 | 5.30 | 5.30 |
| Osmolarity / mOsm/kg | 304 | 304 | 306 | 304 | 307 |
| assay of atropine sulfate / % | 102.2 | 99.8 | 99.3 | 98.1 | 94,6 |
| Impurity C (tropic acid) / % | 0 | 0 | 0 | 0 | 3.17 |
| Total impurities / % | 0,12 | 0 | 1.60 | 2.71 | 3.68 |

**TABLE 5**

| **Example 2C - 40 °C ± 2 °C / 75% ± 5% RH** | | | | | |
|---|---|---|---|---|---|
| | initial | 1 month | 2 months | 3 months | 6 months |
| pH | 4.40 | 4.50 | 4.50 | 4.40 | 4.50 |
| osmolarity | 301 | 301 | 302 | 302 | 307 |
| Assay of atropine sulfate / % of w/w | 101.2 | 99.8 | 100.2 | 102.0 | 100,1 |
| Impurity C (tropic acid) / % of w/v | 0 | 0.15 | 0.28 | 0.42 | 0.85 |
| Total impurities / % of w/v | 0 | 0.15 | 0.28 | 0.69 | 1.64 |

**TABLE 6**

| **Example 2D - 40 °C ± 2 °C / 75% ± 5% RH** | | | | | |
|---|---|---|---|---|---|
| | initial | 1 month | 2 months | 3 months | 6 months |
| pH | 4.3 | 4.3 | 4.3 | 4.3 | 4.3 |
| osmolarity | 290 | 293 | 294 | 296 | 295 |
| Assay of atropine sulfate / % of w/w | 102.8 | 104.7 | 99.2 | 98.5 | 97.8 |
| Impurity C (tropic acid) / % of w/v | 0 | 0.1 | 0.25 | 0.49 | 0.93 |
| Total impurities / % of w/v | 0 | 0.1 | 0.25 | 0.98 | 1.84 |

No significant changes in a pH value and osmolarity for all Examples through the study durations were observed. Example 2A possessed very low stability and decrease in assay of atropine sulfate equalled 15,8% after 1 month at 40°C/75%RH. In turn, surprisingly better results have been found for the Examples 2B, 2C and 2D and the assay of atropine as well as the total level of impurities are within a tolerance limit for the ophthalmic compositions through the study durations.

### Example 3. Impact of the presence of a complexing agent and a viscosity agent on the stability of buffered atropine sulfate formulations

Solutions 3A and 3B were prepared analogously as disclosed in example 1, using the ingredients of Table 7. In the case of Example 3B the sterile filtration through 0.2 microns filter was not carried out.

**TABLE 7**

| **Ingredient** | **Example 3A** | **Example 3B** |
|---|---|---|
| | **% w/w** | **% w/w** |
| Atropine sulfate | 0.01 | 0.01 |
| Sodium chloride | 0.44 | 0.44 |
| Sodium citrate dihydrate | 1.44 | 1.44 |
| Citrate acid monohydrate | 0.31 | 0.31 |
| Disodium edetate | 0.01 | 0.01 |
| hydroxypropyl-β-cyclodextrin | 0.022 | - |
| Hydroxyethylcellulose | - | 0.25 |
| Water for injections | Up to 1 mL | Up to 1 mL |

The pharmaceutical compositions of Table 7 were subjected to accelerated stability studies at 40 °C ± 2 °C / 75% ± 5% RH as per the International Committee on Harmonization stability conditions for 6 months. Samples were tested after 1, 2, 3 and 6 months. The results are shown in Tables 8-9.

**TABLE 8**

| **Example 3A - 40 °C ± 2 °C / 75% ± 5% RH** | | | | | |
|---|---|---|---|---|---|
| | initial | 1 month | 2 months | 3 months | 6 months |
| pH | 5.30 | 5.30 | 5.30 | 5.20 | 5.30 |
| Osmolarity / mOsm/kg | 304 | 304 | 304 | 304 | 307 |
| Assay of atropine sulfate / % of w/w | 101.1 | 98.7 | 98.2 | 97.9 | 93.4 |
| Impurity C (tropic acid) / % of w/v | 0 | 0.61 | 1.19 | 1.71 | 3.38 |
| Total impurities / % of w/v | 0 | 0.61 | 1,19 | 1.93 | 4.23 |

**TABLE 9**

| **Example 3B - 40 °C ± 2 °C / 75% ± 5% RH** | | | | | |
|---|---|---|---|---|---|
| | initial | 1 month | 2 months | 3 months | 6 months |
| pH | 5.40 | 5.40 | 5.30 | 5.30 | 5.30 |
| Osmolarity / mOsm/kg | 308 | 306 | 309 | 306 | 309 |
| Assay of atropine sulfate / % of w/w | 101.7 | 98.1 | 98.1 | 98.1 | 92.9 |
| Impurity C (tropic acid) / % of w/v | 0 | 0 | 0.12 | 0.38 | 3.42 |
| Total impurities / % of w/v | 0 | 0 | 0.12 | 0.62 | 4.36 |

The pH value and osmolality of each of Examples 3A and 3B remained relatively unchanged, within acceptable levels in terms of stability. Both formulations were also good in terms of atropine assay and the total level of impurities.

## Claims

1. A preservative free ophthalmic low concentration atropine sulfate composition consisting of
a) atropine sulfate in an amount from about 0.005 w/w% to about 0.05 w/w%
b) citrate buffer in an amount of less than about 100mM, preferably about 10 mM
c) a tonicity agent
d) water
e) optionally a pH adjuster,
f) optionally a chelating agent
g) optionally a complexing agent, and
h) optionally a viscosity agent
**characterized in that** it has a pH from about 4.0 to about 5.5.

2. The preservative free ophthalmic low concentration atropine sulfate composition according to claim 1, **characterized in that** the pH is from about 4.0 to about 5.0, preferably from about 4.0 to about 4.5, preferably 4.1 to about 4.9, preferably from about 4.2 to about 4.8, preferably from about 4.3 to about 4.7, preferably from about 4.4 to about 4.6, more preferably about 4.5, more preferably 4.5.

3. The preservative free ophthalmic low concentration atropine sulfate composition according to claims 1 or 2, **characterized in that** the molar ratio between citric acid and sodium citrate is from about 0.2 to about 1.4, preferably from about 0.4 to about 1.2, more preferably from about 0.6 to about 1.0, more preferably from about 0.7 to about 0.9, preferably from about 0.80 to about 0.85, more preferably about 0.82.

4. The preservative free ophthalmic low concentration atropine sulfate composition according to claims 1 to 3, **characterized in that** the concentration of the citrate buffer is in an amount of less than about 90 mM, preferably less than about 65 mM, preferably less than about 30mM, more preferably about 10 mM or less, still more preferably about 10mM.

5. The preservative free ophthalmic low concentration atropine sulfate composition according to any one of claims 1 to 4, **characterized in that** the pH adjuster is hydrochloric acid.

6. The preservative free ophthalmic low concentration atropine sulfate composition according to any one of claims 1 to 5, **characterized in that** the chelating agent is EDTA.

7. The preservative free ophthalmic low concentration atropine sulfate composition according to any one of claims 1 to 6, **characterized in that** the tonicity agent is sodium chloride.

8. The preservative free ophthalmic low concentration atropine sulfate composition according to any one of claims 1 to 7, **characterized in that** the complexing agent is cyclodextrin compound, preferably β-cydodextrin, γ- cyclodextrin, hydroxypropyl-β-cyclodextrin or sulfobutylether β-cydodextrin, more preferably hydroxypropyl-β-cyclodextrin.

9. The preservative free ophthalmic low concentration atropine sulfate composition according to any one of claims 1 to 8, **characterized in that** amount of atropine sulfate is about 0.01 wt% or about 0.05wt%.

10. The preservative free ophthalmic low concentration atropine sulfate composition according to any one of claims 1 to 9 consisting of
k) atropine sulfate in an amount of about 0.01 w/w% or about 0.05 w/w%
l) citrate buffer in an amount of less than about 100mM, preferably about 10 mM m) sodium chloride as tonicity agent
n) water, and
o) optionally a chelating agent, preferably EDTA
**characterized in that** it has a pH from about 4.0 to about 5.5.

11. The preservative free ophthalmic low concentration atropine sulfate composition according to any one of claims 1 to 10 consisting of
f) atropine sulfate in an amount of about 0.01 w/w% or about 0.05 w/w%
g) citrate buffer in an amount of less than about 100mM, preferably about 10 mM
h) sodium chloride as tonicity agent
i) water, and
j) optionally a chelating agent, preferably EDTA
**characterized in that** it has a pH about 4.5.

12. A process for the preparation of the preservative free ophthalmic low concentration atropine sulfate composition according to any one of claims 1 to 11, **characterized in that** it comprises the following steps:
i) dissolve all the components except atropine sulfate in water
ii) dissolve the atropine sulfate in the solution obtained in step i)
iii) sterile filtration of the solution obtained in step ii)
iv) dispense the filtered solution obtained in step iii) to a sterile container, and
v) aseptic closure of the container obtained in step iv) by a sterile dropper or dropper system suitable for ophthalmic compositions.

13. A pharmaceutical dosage form comprising preservative free ophthalmic low concentration atropine sulfate composition according to any one of claims 1 to 11, preferably in the form of a solution.

14. The preservative free ophthalmic low concentration atropine sulfate composition according to any one of claims 1 to 11 or the pharmaceutical dosage form according to claim 13 for use in therapy, preferably, for use in the treatment and/or prevention of myopia, preferably in a paediatric population.

15. A method for treating and/or preventing myopia in a patient in need thereof, preferably in a paediatric population, comprising the step of administering therapeutically effective amount of the preservative free ophthalmic low concentration atropine sulfate composition according to any one of claims 1 to 11 or the pharmaceutical dosage form according to claim 13.
